# EUROPEAN PATENT SPECIFICATION

(11) **EP 4 498 898 B1**
(45) Date of publication and mention of the grant of the patent: **18.11.2026**
(21) Application number: 23717422.2
(22) Date of filing: 30.03.2023
(51) Int. Cl.: A61B 5/00

(54) **LIGHT GUIDE MODULE AND APPARATUS FOR MEASURING AN OPTICAL PROPERTY OF A MEDIUM**
LICHTLEITERMODUL UND VORRICHTUNG ZUR MESSUNG EINER OPTISCHEN EIGENSCHAFT EINES MEDIUMS
MODULE DE GUIDAGE DE LUMIÈRE ET APPAREIL DE MESURE D'UNE PROPRIÉTÉ OPTIQUE D'UN MILIEU

(30) Priority: 31.03.2022 GB 202204719
(43) Date of publication of application: 05.02.2025
(73) Proprietor: Gowerlabs Limited, Penge, London SE20 7BW (GB)
(72) Inventor: EVERDELL, Nicholas Lyndon, London SE20 7BW (GB); POWELL, Samuel, London SE20 7BW (GB); SMITH, Gregory Culver, London SE20 7BW (GB)
(74) Representative: Openshaw & Co.
(86) International application number: PCT/EP2023/058290
(87) International publication number: WO 2023/187054

(56) References cited:
- US-A- 4 768 858
- US-A1- 2019 175 068
- US-A1- 2021 259 632

## Description

### Technical Field

The present disclosure relates to a light guide module for use with apparatus for measuring an optical property of a medium and to an apparatus for measuring an optical property of a medium.

### Background

There are many applications in which it is useful to be able to measure an optical property of a medium. A particular example of the optical property is changes in concentration of a chromophore in a biological medium. Other examples include the absorption coefficient and the decorrelation rate. Known apparatus tends to be bulky and uncomfortable for the subject in the case the medium is a biological one. Known apparatus is also subject to low levels of signal-to-noise, particularly because the signal strength is often very low whilst noise levels can be very high, which can mask the signals that are to be obtained.

This is a problem in apparatus that uses transmission of light to and from the subject, and is a particular problem in the case that the apparatus is being used to transmit light into the head of a (human) subject and detect light that is diffusely reflected back out.

In our WO2018/033751 A1, there is disclosed a measuring apparatus for fitting to an animal body for measuring changes in concentration of a chromophore in the animal body. The measuring apparatus has a plurality of devices, which may be referred to colloquially as "tiles". Each device or tile has at least one light source for emitting light towards an animal body and at least one light detector for detecting light returning from the animal body. A device controller in the tile receives signals from and sends signals to a main controller via a connection arrangement, which may be or include a shared bus. Light guides, in the form of optical fibres, are provided to help channel the light from the light source(s) to the tissue under investigation and back from the tissue to the light detector(s).

US4768858A discloses a fiberoptic which includes a guide for light or other electromagnetic energy which has a single fiber core with a ring-shaped cross-section and defines an outer cylindrical surface and an inner cylindrical surface. A cylindrical central hollow channel extends between the ends of the fiberoptic through the center of the single fiber ring-shaped core. Various devices including coherent fiber viewing bundles, hollow tubular transport channels and the like may be slidingly disposed through the center channel of the core.

US20211259632 A1 discloses a wearable module for use in an optical measurement system which includes a housing including a top surface, a light guide including a distal end portion adapted to protrude from the top surface of the housing, and a spring member configured to bias the distal end portion of the light guide away from the top surface of the housing. The light guide is configured to receive, at the distal end portion, photons from a light pulse scattered by a target within a body of a user and guide the received photons toward a photodetector.

### Summary

According to a first aspect disclosed herein, there is provided a light guide module for use with an apparatus which fits to an animal body for measuring an optical property of a medium in the animal body, the light guide module comprising:
a support member;
a first light guide supported in the support member; and
a second light guide supported in the support member;
wherein one of the first light guide and the second light guide is a fixed light guide which is arranged to be fixed relative to the support member, and wherein the other of the first light guide and the second light guide is a movable light guide which is arranged to be movable back and forth relative to the support member between an extended position and a retracted position;
wherein the second light guide is arranged coaxially with the first light guide so that light can pass between the first light guide and the second light guide;
wherein at least one of the fixed light guide and the movable light guide is a hollow tube light guide having a hollow interior which receives the other light guide;
the interior of said hollow tube light guide being reflective such that when the movable light guide is in the extended position, at least one of: (i) light entering an end of said hollow tube light guide can be internally reflected by the interior of said hollow tube light guide into an end of the other light guide that is facing the other end of said hollow tube light guide, and (ii) light exiting said end of the other light guide that is facing the other end of said hollow tube light guide can be internally reflected by the interior of said hollow tube light guide towards said end of said hollow tube light guide.

In some examples, the hollow light guide is the fixed light guide. In other examples, the hollow light guide is the movable light guide. In some examples, one of the light guides is a hollow tube (which is fixed or movable in different examples) and the other light guide is a solid light guide (which is movable or fixed in different examples). In other examples, both the first light guide and the second light guide are hollow tubes, with one of them being fixed and the other being movable. In any case, as the movable light guide moves back and forth, there is a telescopic like action between the light guides which accommodates movement of the movable light guide.

When provided in an apparatus for measuring an optical property of a medium in the animal body, the movable light guide can move in and out as the apparatus is fitted to and moved over the animal body so that a good contact is made between the first end of the movable light guide and the adjacent part of the animal body. This ensures efficient transfer of light from the movable light guide to the animal body and/or from the animal body into the movable light guide. The telescopic arrangement of the two light guides and the reflective interior of the hollow tube light guide accommodates the movement whilst maintaining a good transfer of light between the light guides. The use of coaxial, telescopic light guides means that the overall cross-sectional area or "footprint" of the light transmission parts is small.

In an example, the fit between the hollow interior of said hollow tube light guide which receives the other light guide and the other light guide is a clearance fit.

This helps reduce light losses whilst still allowing the light guides to move relative to each other. The clearance fit may in particular be a sliding fit.

In an example, said hollow tube light guide is the fixed light guide and is fixed to and supported by the support member or integral with the support member so as to be fixed against movement relative to the support member.

In an example, said hollow tube light guide is the movable light guide.

In an example, one of the light guides is said hollow tube light guide and the other light guide is a solid optical fibre or bundle of solid optical fibres.

In an example, both the first light guide and the second light guide are hollow tube light guides each having a reflective interior, wherein one of the hollow light guides is the movable light guide which is movable in and out of the hollow interior of the other, fixed, light guide.

In an example, the light guide module comprises a biasing arrangement to bias the movable light guide in a direction away from the fixed light guide.

In use, the biasing arrangement biases the movable light guide in a direction towards the subject animal body, which helps to maintain a good contact between the end of the movable light guide and the surface of the subject.

In an example, the biasing arrangement comprises a spring arranged to bias the movable light guide in a direction away from the fixed light guide.

In an example, the biasing arrangement comprises a first magnet fixed in relation to the movable light guide and a second magnet fixed in relation to the fixed light guide, the magnets being arranged to repel each other so as to bias the movable light guide in a direction away from the fixed light guide.

In an example, the biasing arrangement is provided by a volute spring having a plurality of coils, at least one of the first light guide and the second light guide being provided by at least one coil of the volute spring. In an example, the first light guide and the second light guide are provided by respective coils of the volute spring.

In an example, the light guide module comprises:
plural sets of first and second light guides supported in the support member;
wherein for each set of first and second light guides:
   one of the first light guide and the second light guide is a fixed light guide which is arranged to be fixed relative to the support member, and wherein the other of the first light guide and the second light guide is a movable light guide which is arranged to be movable back and forth relative to the support member between an extended position and a retracted position;
   the second light guide is arranged coaxially with the first light guide so that light can pass between the first light guide and the second light guide;
   at least one of the fixed light guide and the movable light guide is a hollow tube light guide having a hollow interior which receives the other light guide; and
   the interior of said hollow tube light guide being reflective such that when the movable light guide is in the extended position, at least one of: (i) light entering an end of said hollow tube light guide can be internally reflected by the interior of said hollow tube light guide into an end of the other light guide that is facing the other end of said hollow tube light guide, and (ii) light exiting said end of the other light guide that is facing the other end of said hollow tube light guide can be internally reflected by the interior of said hollow tube light guide towards said end of said hollow tube light guide.

In an example, each of the movable light guides is movable back and forth relative to the support member independently of the other movable light guides.

According to a second aspect disclosed herein, there is provided an apparatus for fitting to an animal body for measuring an optical property of a medium in the animal body, the apparatus comprising:
at least one light source for emitting light towards an animal body to which the measuring apparatus is fitted in use;
at least one light detector for detecting light returning from said animal body;
and a light guide module as described above.

In an example, the fit between the hollow interior of said hollow tube light guide which receives the other light guide and the other light guide is a clearance fit.

In an example, the apparatus comprises a biasing arrangement to bias the movable light guide in a direction away from the fixed light guide.

In an example, the biasing arrangement is provided by a volute spring having a plurality of coils, at least one of the first light guide and the second light guide being provided by at least one coil of the volute spring.

In an example, said hollow tube light guide is the fixed light guide and is fixed to and supported by the support member or integral with the support member so as to be fixed against movement relative to the support member.

In an example, said hollow tube light guide is the movable light guide.

In an example, one of the light guides is said hollow tube light guide and the other light guide is a solid optical fibre or bundle of solid optical fibres.

In an example, both the first light guide and the second light guide are hollow tube light guides each having a reflective interior, wherein one of the hollow light guides is the movable light guide which is movable in and out of the hollow interior of the other, fixed, light guide.

In an example, the apparatus comprises plural sets of fixed and movable light guides, wherein each set of fixed and movable light guides is associated with a respective one of the light source(s) and the light detector(s).

In an example, each of the movable light guides is movable back and forth relative to the support member independently of the other movable light guides.

### Brief Description of the Drawings

To assist understanding of the present disclosure and to show how embodiments may be put into effect, reference is made by way of example to the accompanying drawings in which:
Figure 1 shows a schematic side view of an assembly of a known device, a dock for the device, and a light guide arrangement for the device as described in our WO2018/033751A1;
Figure 2 shows a schematic side view of a known carrier having a plurality of docks into which devices can be removably located, as described in our WO2018/033751A1;
Figures 3A, 3B and 3C respectively show schematic perspective views of the known device, dock and light guide arrangement of the assembly of Figure 1;
Figures 4A and 4B respectively show schematic perspective views of a device and a dock, and Figure 4C shows a perspective view of a first example of a light guide module according to an aspect of the present disclosure;
Figure 5 shows a schematic side view of an assembly of the device, the dock and the first example of the light guide module of Figures 4 in a first configuration;
Figure 6 shows a schematic side view of the assembly of the device, the dock and the first example of the light guide module of Figures 4 in a second configuration;
Figures 7A, 7B and 7C respectively show a schematic perspective view, side view and cross-sectional view of the first example of the light guide module of Figure 4C in the first configuration;
Figures 8A, 8B and 8C respectively show a schematic perspective view, side view and cross-sectional view of the first example of the light guide module of Figure 4C in the second configuration;
Figure 9 shows a schematic perspective view of an example of a board on which one or more light sources and light detectors of the device of Figure 4A are mounted;
Figures 10A and 10B show cross-sectional views of another example of a light guide module according to the present disclosure in the first and second configuration respectively;
Figures 11A and 11B show cross-sectional views of another example of a light guide module according to the present disclosure in the first and second configuration respectively;
Figures 12A and 12B show cross-sectional views of another example of a light guide module according to the present disclosure in the first and second configuration respectively;
Figures 13A and 13B show cross-sectional views of another example of a light guide module according to the present disclosure in the first and second configuration respectively;
Figures 14 to 17 show cross-sectional views of further examples of a light guide module according to the present disclosure;
Figure 18 shows a cross-sectional view of another example of a light guide module according to the present disclosure; and
Figure 19 shows a cross-sectional view of another example of a light guide module according to the present disclosure.

### Detailed Description

As mentioned above, there are many applications in which it is useful to be able to measure an optical property of a biological medium. For example, it can be useful to measure changes in concentration of a chromophore in an animal body, including in particular a human body, where chromophores may be found, for example, in blood flowing through the brain, or heart, or skeletal muscle, etc., etc. Other example properties include absorption coefficient and decorrelation rate.

As one particular example, apparatus for performing functional neuroimaging which aim to measure regional brain activity are known. Functional near-infrared spectroscopy (fNIRS) is a known non-invasive technique for providing information on the vascular and metabolic response to brain activity. The chromophores may be or include for example oxy-haemoglobin, deoxy-haemoglobin and oxidised cytochrome c oxidase. Oxy-haemoglobin and deoxy-haemoglobin are found in the blood. Oxidised cytochrome c oxidase can be found in tissue. Measurement of the changes in concentration of these chromophores may offer insight into, for example, regions of activity in the brain.

In such apparatus, light, which is infrared in the case of fNIRS, is emitted at the subject by one or more light sources and light reflected or transmitted back from the subject is detected by one or more light detectors. Light guides may be provided, typically one light guide for each light source and each light detector, in order to maximise the coupling of light from the light sources to the subject and from the subject to the light detectors, and thereby improve the signal-to-noise ratio. The light guides may be in the form of or provided by optical fibres.

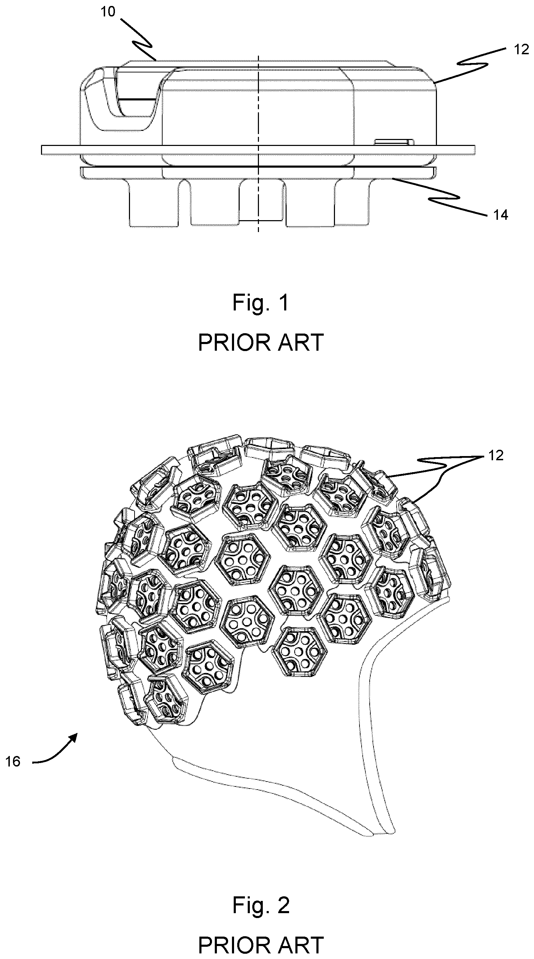

A specific arrangement for such apparatus is disclosed in our WO2018/033751 A1. Figures 1 to 3 are taken from our WO2018/033751A1 and will be briefly discussed as useful background.

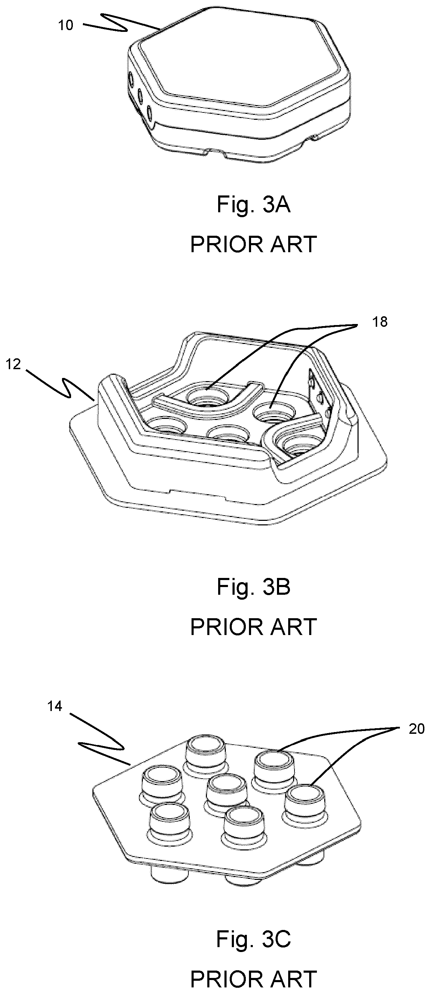

Figure 1 shows a schematic side view of an assembly of a device 10, a dock 12 for the device 10, and a light guide arrangement 14 for the device 10 as described in our WO2018/033751A1. Figure 2 shows a schematic side view of a carrier 16 having a plurality of docks 12 into which devices 10 can be removably located as described in our WO2018/033751A1. Figures 3A, 3B and 3C respectively show schematic perspective views of the known device 10, dock 12 and light guide arrangement 14 of the assembly of Figure 1. The carrier 16 in this case is in the form of a cap or other headgear to be fitted to the head of a subject. In this case the docks 12 are permanently fixed to the carrier 16 and the devices 10 can be fitted in and removed from the docks 12, which allows the number of and configuration of the devices 10 over the carrier 16 (and therefore over the subject's head) to be easily varied as desired. In practice, there may be a large number of docks 12 over the carrier 16 and all or only some of them may be filled with devices 10 as necessary or desirable for the particular study of the subject that is being undertaken.

One or more light sources and one or more light detectors are mounted in the device 10. The dock 12 removably receives a device 10 and acts as a mechanical support and retainer for the device 10. The dock 12 and the device 10 may be a friction fit or a snap fit, with ridges and recesses, etc. to secure the device 10 in the dock 12. The dock 12 in this example also provides power and data in and data out connections for the device 10.

The dock 12 has plural through holes 18, for example, one through hole 18 for each light source and for each light detector, so that light can pass through the dock 12 between the subject and the light source(s) and the light detector(s) respectively.

Referring particularly to Figure 3C, the light guide arrangement 14 has plural light guides 20. There is typically one light guide 20 for each light source and for each light detector. The light guides 20 therefore typically correspond in number and location to the through holes 18 of the dock 12. The light guides 20 help channel the light from the light source(s) to the subject's tissue under investigation and back from the subject to the light detector(s). This helps to improve the signal-to-noise ratio by reducing the amount of light that is lost between the tissue and the device 10.

The use of such light guides 20 is of particular advantage when it is the subject's head, and particular the brain, that is being subject to study, particularly in the case of fNIRS which uses infrared light. This is because hair is a strong absorber of light, and particularly a strong absorber of near-infrared light, and is often a major factor in preventing a measurement with a good signal-to-noise ratio being obtained. The light guides 20 comb through the user's hair, i.e. pass between the hair strands, which means that the light to and from the light sources and light detectors is less likely to be absorbed by the hair as the light passes through the light guides 20 instead. This improves the quality of the detected signal.

Ideally, the distal ends of each of the light guides 20 (i.e. the end that is remote from the light source or light detector respectively) are in contact with the subject's scalp or other body part that is being studied in order to minimise the light losses and therefore maximise the signal-to-noise ratio. A problem with light guides that are static is that they cannot conform well to the shape of the subject's scalp or other body part that is being studied. Ridges or bumps or depressions in the scalp or other body part often mean that whilst some light guides are making good contact, others of the light guides are held away from the scalp or other body part. This is a particular problem in the case that there are many light guides per unit area, which is required for a high density study and high resolution imaging, such as is often desired in the case of fNIRS.

A known solution to this is to allow one or more or all of the light guides to move back and forth, and particularly to move back and forth independently of the others. Examples of this are disclosed in WO2011/083563A1, US2015/0038811A1 and US2021/0259632A1. Each of these documents discloses apparatus for optical study and imaging, including for fNIRS of the brain, in which light guides are used to convey light to and from the subject and in which the light guides are movable. The movement of the light guides allows the light guides to conform to the shape of the body part better, which helps to make a good contact between the light guides and the subject's body. In each of these documents, the movable light guides are springloaded so as to be biased towards contact with the subject's body to assist in ensuring a good contact with the subject's body.

However, with movable light guides, particularly in the case of arrangements like those disclosed in US2021/0259632A1 in which the light guides move relative to the light sources or light detectors respectively, there remains a problem in ensuring efficient transmission of light out of the light sources into the light guides and out of the light guides into the light detectors over the whole movement range of the light guides. In particular, when the light guide has been moved away from the light source or light detector respectively, light can be lost between the light guide and the light source or light detector respectively. The device disclosed in US2021/0259632A1 attempts to solve this on the light source side by the use of a prism and lens plate, located between the end of the light guide and the light source, for directing and focussing the light onto the end of the light guide. The device disclosed in US2021/0259632A1 attempts to solve this on the light detector side by the use of first and second lenses, located between the end of the light guide and the light detector, for collimating and focussing the light on to the light detector.

In examples described herein, there is provided a light guide module for use with an apparatus which fits to an animal body for measuring an optical property of a medium in the animal body. Examples of and applications of such apparatus are described further above. In one example, the light guide module has a support member, a first light guide and a second light guide supported in the support member. One of the light guides is a fixed light guide and the other light guide is a movable light guide which is movable back and forth relative to the support member between an extended position and a retracted position. The light guides are arranged coaxially. At least one of the fixed light guide and the movable light guide is a hollow tube light guide having a hollow interior which receives the other light guide. The interior of the hollow tube light guide is reflective. As such, when the movable light guide is in the extended position, at least one of: (i) light entering an end of said hollow tube light guide can be internally reflected by the interior of said hollow tube light guide into an end of the other light guide that is facing said end of said hollow tube light guide, and (ii) light exiting said end of the other light guide can be internally reflected by the interior of said hollow tube light guide towards said end of said hollow tube light guide.

This ensures efficient transfer of light from the light source to the animal body and/or from the animal body to the light detector. The hollow tube light guide (which in some examples is the fixed light guide and in other examples is the movable light guide) having a reflective interior accommodates movement of the movable light guide whilst maintaining a good transfer of light.

This arrangement maximises the coupling of light from the light sources to the subject and from the subject to the light detectors, even though the movable light guide has moved away from the light source or light detector respectively. This therefore reduces losses of the light passing from the light sources to the subject and/or from the subject to the light detector. Furthermore, the hollow tube light guide can also prevent ambient light from entering the system, which can otherwise worsen the signal-to-noise ratio. Moreover, the use of a hollow tube light guide for accommodating movement of the movable light guide means that collimating and focussing lenses and the like used in some known apparatus to direct light into and out of a movable light guide can be avoided. This in turn means that the overall cross-sectional area or "footprint" of the light transmission parts is small. This allows a higher density (i.e. number per unit area) of light sources and light detectors to be provided, in turn enabling higher quality and resolution to be obtained. Alternatively or additionally, this means that the spacing between adjacent light guides can be relatively large, making it easier to comb the light guides through a subject's hair when fitting the apparatus to the subject's head and moving the apparatus and light guides over the head. In addition, reducing the light losses means that the light guides themselves can have a smaller diameter, which again makes it easier to comb the light guides through a subject's hair when fitting and locating the apparatus to the subject's head. Finally, avoiding the use of collimating and focussing lenses and the like also means that the number of interfaces through which the light must pass between the light sources and subject and the light detector can be minimised (indeed, can be unchanged from the arrangement as disclosed in our WO2018/033751A1 in some examples). This keeps down any reflective losses that can occur when light passes through an interface between materials having different refractive indices, again improving the signal-to-noise ratio.

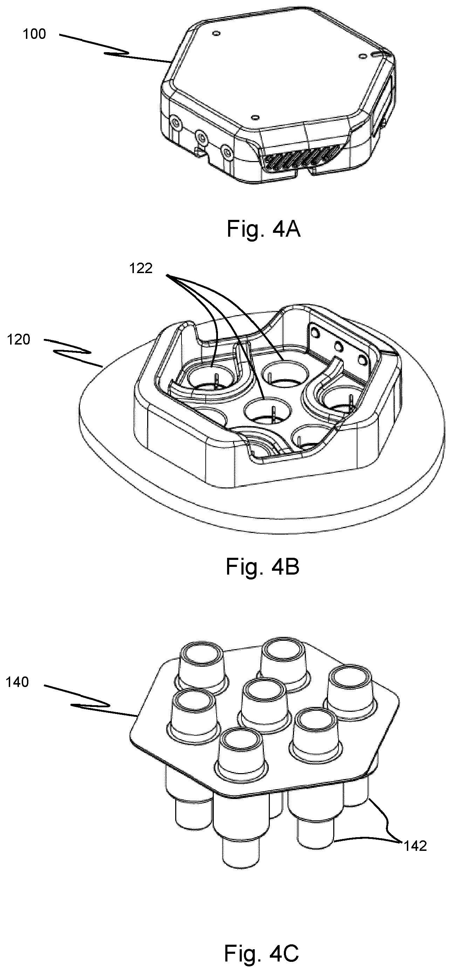

Some specific examples of the present disclosure will now be described with reference to Figures 4 to 17. It should be noted that there are numerous parts that are the same or that correspond for the various examples and the description thereof is not repeated throughout for reasons of brevity. Likewise, not all reference numerals are repeated across the various figures for reasons of clarity.

Referring first to Figure 4, Figures 4A and 4B respectively show schematic perspective views of a device 100 and a dock 120, and Figure 4C shows a perspective view of a first example of a light guide module 140 according to an aspect of the present disclosure. The device 100 and the dock 120 may for example generally be of the type discussed more fully in our WO2018/033751A1.

In this example, the device 100 can be removably located in the dock 120. The dock 120 removably receives the device 100 and acts as a mechanical support and retainer for the device 100. The dock 120 and the device 100 may be a friction fit or a snap fit, with ridges and recesses, etc. to secure the device 100 in the dock 120. The dock 120 in an example also provides power and data connections for the device 100. In an example, plural such docks 120 with associated light guide modules 140 may be permanently fixed to a carrier such as for example a cap or other headgear as mentioned above (see also Figure 2) and discussed more fully in our WO2018/033751A1. This allows the number of and configuration of the devices 100 over the carrier (and therefore over the subject's head) to be easily varied by clipping them in and out of docks 120 as desired.

As an alternative, the device(s) 100 and the light guide module(s) 140 may be "permanently" fixed to or be part of a carrier such as for example a cap or other headgear, and not to be easily removed and relocated, and docks for allowing the devices 100 to be removably attached are not provided.

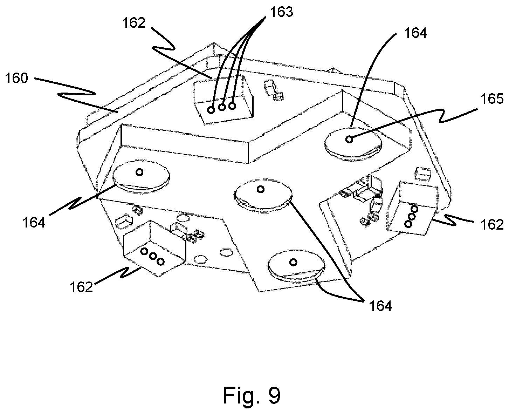

One or more light sources and one or more light detectors are mounted in the device 100. Referring to Figure 9, this shows a schematic perspective view of an example of a board 160 on which one or more light source units 162 and light detector units 164 of the device 100 are located. The board 160 in this example is provided in and as a component of the device 100. In the specific example shown, the board 160 has three light source units 162 and four light detector units 164. The three light source units 162 are symmetrically arranged on the board 160, in this example being located towards alternating corners of the hexagon. The four light detector units 164 are likewise symmetrically arranged on the board 160, in this example with three being located towards the other alternating corners of the hexagon and the fourth being located centrally of the hexagon.

Each light source unit 162 has or contains at least one light source 163 for emitting light. In an example a light source unit 162 has a plurality of light sources 163. In the specific example shown, each light source unit 162 has three light sources 163. Each light source 163 may emit light having a wavelength in the range of for example around 600 nm to 1000 nm. The light sources 163 may emit light having different wavelengths. That is, in an example, each light source unit 162 has a first light source 163 which can emit light at a first wavelength, a second light source 163 which can emit light at a second wavelength, and a third light source 163 which can emit light at a third wavelength, each wavelength being different. In an example, the three wavelengths might be 735 nanometres, 810 nanometres and 850 nanometres. The light sources 163 may be for example light-emitting diodes (LEDs). In use in an example, each of the light sources 163 is powered in turn such that, at least within a particular light source unit 162, only one light source 163 is emitting light at a time.

Each light detector unit 164 has at least one light detector 165, which may be for example a photodiode, for detecting light. In the example shown, each light detector unit 164 has a single light detector 165. The light detectors 165 are arranged to detect light having a wavelength that is emitted by the light sources 163. In use in an example, each light detector 165 is continually able to detect light.

The board 160 may be a printed circuit board (PCB). In the example shown, the light source units 162 and light detector units 164, and specifically the light sources 163 and light detectors 165 contained therein, are mounted directly on the board 160. An advantage of mounting the light source units 162 and light detector units 164, and specifically the light sources 163 and light detectors 165 contained therein, directly on the PCB 160 is that it avoids (relatively) long connecting wires from the PCB to the light sources 163 and light detectors 165, which therefore reduces noise and interference. The light detector units 164 are arranged on the PCB 160 close to respective amplifier and other circuits (not shown), which again helps to reduce noise arising in the device 100.

Returning to the dock 120 briefly, in this example, this has seven through holes 122, one for each of the light source units 162 and light detector units 164 of the device 100, for light to pass through. When the dock 120 and light guide module 140 are assembled, parts of the light guide module 140 project through the through holes 122 of the dock 120 to transmit the light, as will be discussed further below.

Discussing now the first example of the light guide module 140 in more detail, a function of this is to be or provide a support member to support one or more light guides 142 which in use are close to and preferably in contact with the animal body, for example the scalp of the subject's head. As noted, the light guide module 140 may be provided as a discrete component or may be part of a larger carrier, such as for example a cap or other headgear, which is fitted to the animal body in use.

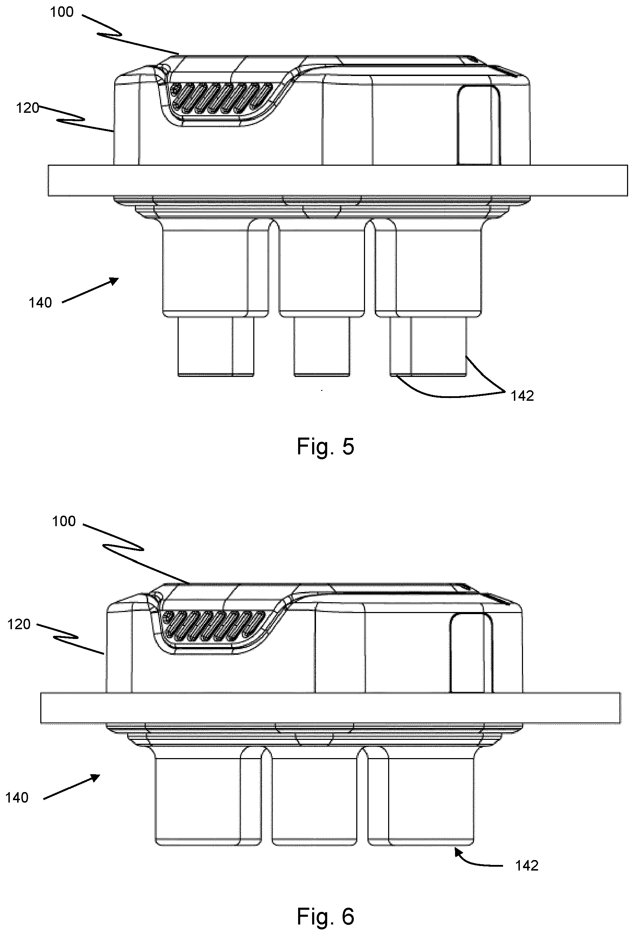

The light guides 142 are arranged to be able to move back and forth, i.e. in a reciprocating manner, in and out, relative to the light sources 163 and the light detectors 165 of the device 100. That is, in this example where a separate light guide module 140 is provided to support the light guides 142, the light guides 142 can move back and forth relative to the body of the light guide module 140. This is illustrated in Figure 5 which shows the light guides 142 in an extended configuration, that is, most distant from the light sources 163 and the light detectors 165 of the device 100; and in Figure 6 which shows the light guides 142 in a retracted configuration, that is, closest to the light sources 163 and the light detectors 165 of the device 100. This movement of the light guides 142 in and out enables the distal ends 144 of the light guides 142 (i.e. the end that is remote from the light source 163 or light detector 165 respectively) to contact the subject's scalp or other body part that is being studied, as it allows recesses and ridges and the like in the scalp or other body part to be accommodated. Maintaining contact between the distal ends 144 of the light guides 142 with the body part minimises the light losses and therefore maximises the signal-to-noise ratio.

A problem with allowing the light guides 142 to move back and forth relative to the light sources 163 and the light detectors 165 of the device 100 is that the proximal ends 146 of the light guides 142, that is, the ends of the light guides 142 that are closest to the light sources 163 and the light detectors 165, can become located some distance from the light sources 163 and the light detectors 165. This means that light can be lost between the light guide 142 and the light source 163 or light detector 165 respectively. It also means that ambient light may enter into the transmission path, which can affect both the illumination of the subject and the quality of the returning light signal that is detected.

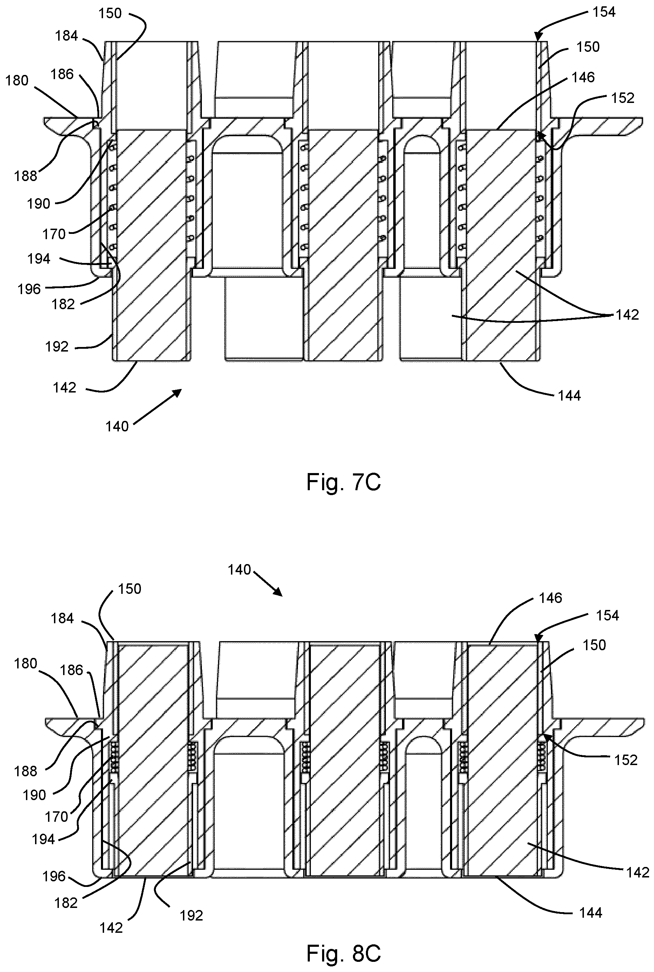

To address this, and as shown most clearly in the cross-sectional views of Figure 7C and Figure 8C, at least one and preferably each movable light guide 142 is associated with a respective second light guide 150. The second light guide 150 is fixed relative to the movable light guide 142 (and therefore fixed relative to the light guide module 140 in this example). The first, movable light guides 142, which in this example in use are close to and preferably in contact with the animal body, may be referred to as "distal" light guides 142 as they are remote from the respective light sources 163 and light detectors 165 of the device 100. The second, fixed light guides 150, which in this example are close to the respective light source 163 or light detector 165, may be referred to as "proximal" light guides 150.

In the example shown in Figure 7C and Figure 8C, each of the fixed light guides 150 is a hollow tube, which is arranged coaxially with the respective movable light guide 142. The hollow tube fixed light guide 150 has a first, distal, end 152, which is furthest from the light source 163 or the light detector 165 respectively (and therefore closest to the subject in use), and a second, proximal, end 154, which is closest to the light source 163 or the light detector 165 respectively. As the movable light guide 142 moves back and forth (for example, as the movable light guides 142 are moved over the subject's body), the movable light guide 142 moves back and forth within the hollow tube light guide 150, in a reciprocating or telescopic manner. The interior surface of the hollow tube light guide 150 is reflective. The interior surface of the hollow tube light guide 150 is preferably highly reflective at least for the light that is emitted by the light source 163. Accordingly, in the case that the movable light guide 142 is for a light source 163, when the movable light guide 142 moves away from the light source 163 (down the hollow tube light guide 150 in Figure 7C), any light emitted by the light source 163 towards the movable light guide 142 that may otherwise be lost is reflected by the interior surface of the hollow tube light guide 150 towards the movable light guide 142. Likewise, in the case that the movable light guide 142 is for a light detector 165, when the movable light guide 142 moves away from the light detector 165 (down the hollow tube light guide 150 in Figure 7C), any light exiting the movable light guide 142 that may otherwise be lost is reflected by the interior surface of the hollow tube light guide 150 towards the light detector 165. This maximises the amount of light passing to and from the movable light guide 142 and the light source 163 and light detector 165 respectively, even though the movable light guide 142 has moved away to be remote from the light source 163 and light detector 165 as the case may be. This helps achieve a good signal to noise ratio.

The fit between the hollow tube light guide and the other light guide that moves within it may be for example a clearance fit, and may particularly be a sliding fit.

In short, providing a second light guide in the form of a hollow tube having a reflective interior and within which the first light guide moves back and forth helps reduce light losses whilst still allowing the first light guide to move to make contact with the subject animal body in use. This therefore maximises the coupling of light from the light sources to the subject and from the subject to the light detectors, even though the movable light guide has moved away from the light source or light detector respectively.

Furthermore, the hollow tube light guide can be opaque, at least to ambient light (including visible light and infrared) which prevents ambient light from entering the system, which can otherwise worsen the signal-to-noise ratio.

In addition, this telescopic arrangement of light guides means that collimating and focussing lenses and the like used in some known apparatus to direct light into and out of a movable light guide can be avoided. This in turn means that the overall cross-sectional area or "footprint" of the light transmission parts is small. This allows a higher density (i.e. number per unit area) of light sources and light detectors to be provided, in turn enabling data of higher quality and resolution to be obtained. Alternatively or additionally, this means that the spacing between adjacent light guides can be relatively large, making it easier to comb the light guides through a subject's hair when fitting the apparatus to the subject's head and moving the apparatus and light guides over the head. In addition, reducing the light losses means that the light guides themselves can have a smaller diameter, which again makes it easier to comb the light guides through a subject's hair when fitting and locating the apparatus to the subject's head. Finally, avoiding the use of collimating and focussing lenses and the like also means that the number of interfaces through which the light must pass between the light sources and subject and the light detectors can be minimised (indeed, can be unchanged from the arrangement as disclosed in our WO2018/033751A1 in some examples). This reduces any reflective losses that can occur when light passes through an interface between materials having different refractive indices, again improving the signal-to-noise ratio.

It may be noted that at one extreme end of its motion, the movable light guide 142 may move entirely out of the hollow tube light guide 150. However, it is preferred that the proximal end 146 of the movable light guide 142, which is closest to the light source 163 or the light detector 165 respectively, always remains within the hollow tube light guide 150 (as indicated in Figure 7C), even when the movable light guide 142 has moved furthest from the light source 163 or light detector as the case may be. This is because this ensures that the maximum amount of light from a light source 163 enters the movable light guide 142, and ensures that the maximum amount of light exiting the movable light guide 142 reaches the light detector 165, respectively, by virtue of the light being reflected by the interior surface of the hollow tube light guide 150.

In this example, there is one set or pair of a movable light guide 142 and a fixed light guide 150 for each of the light source units 162 and light detector units 164 of the device 100. In other examples, not all of the light source units 162 and light detector units 164 of the device 100 have an associated set or pair of a movable light guide 142 and a fixed light guide 150.

The reflective interior surface of the hollow tube light guide 150 may be provided in a number of ways. For example, the whole of the hollow tube light guide 150 may be formed of a reflective material, such as for example a metal, including for example stainless steel. Alternatively or additionally, the interior surface of the hollow tube light guide 150 may be coated with a reflective material. A metallic mirror coating or a dielectric highly reflective coating may be used. Suitable metallic materials include for example aluminium, gold, silver, titanium, etc.

In an example, the movable light guides 142 are biased towards the subject in use, that is away from the light sources 163 and light detectors 165 respectively, namely to the extended configuration. This helps to ensure that the movable light guides 142 make good contact with the subject, or at least bring the distal ends 144 of the movable light guides 142 closer to the subject.

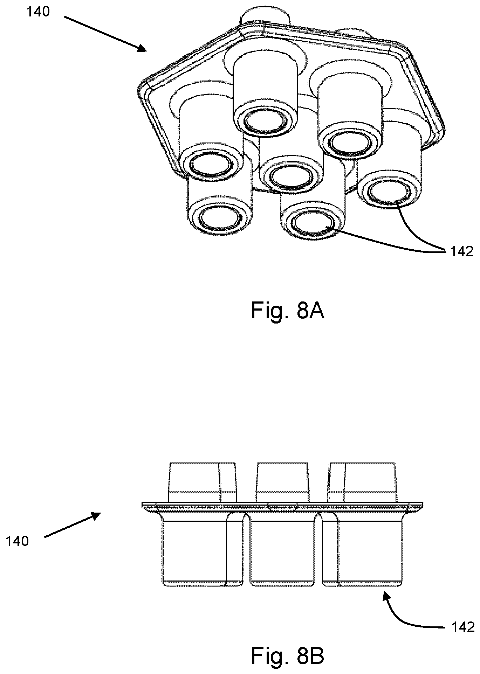

A number of different arrangements for biasing the movable light guides 142 towards the extended configuration are possible. In the example shown in Figure 7C and Figure 8C, the biasing arrangement is provided by one or more springs for each pair of movable light guide 142 and fixed light guide 150. In particular, in the example shown in Figure 7C and Figure 8C, a compression spring 170, in this example a compression coil spring 170, is provided to bias the movable light guide 142 to the extended configuration, namely towards the subject in use.

In more detail, the light guide module 140 has a generally planar base portion 180 and through holes 182 for the first and second light guides 142, 150 to pass through. An elongate tubular collar 184 is located in each through hole 182 to extend therethrough. The upper part of the elongate tubular collar 184 has an external flange 186 around its periphery towards one end. The flange 186 sits in a corresponding recess 188 provided in a facing upper portion of the base portion 180 around the through hole 182 of the base portion 180. The flange 186 may be a snap or press fit in the recess 188 and/or may be fixed in using an adhesive or in some other manner. The fixed light guide 150 is located within the upper part of the elongate tubular collar 184. The fixed light guide 150 may be a snap or press fit in the upper part of the elongate tubular collar 184 and/or may be fixed in using an adhesive or in some other manner. Below the external flange 186 of the elongate tubular collar 184, the elongate tubular collar 184 has an internal flange 190. The elongate tubular collar 184 may be formed of for example black ABS (acrylonitrile butadiene styrene) or some other suitable opaque material.

A second tubular collar 192 is located within the elongate tubular collar 184. The second tubular collar 192 may be formed of for example stainless steel or some other rigid material. The distal end of the movable light guide 142 is fixed within the second tubular collar 192. As the movable light guide 142 moves back and forth relative to the fixed light guide 150, the second tubular collar 192 moves back and forth within the elongate tubular collar 184. In this example, the upper end of the second tubular collar 192 has an external flange 194 around its periphery. The lower part of the module 140 has an internally facing flange 196 towards the lower part of the through hole 182. The external flange 194 of the second tubular collar 192 bears against the internally facing flange 196 at the lower part of the through hole 182 to retain the second tubular collar 192 in the through hole 182 when the movable light guide 142 and the second tubular collar 192 move to the most extended position (downwards in Figure 7C).

The external flange 194 of the second tubular collar 192 faces (is opposed to) the internal flange 190 of the elongate tubular collar 184. The spring 170 is located in the cylindrical space between the movable light guide 142 and the lower part of the elongate tubular collar 184, and is trapped between the external flange 194 of the second tubular collar 192 and the internal flange 190 of the elongate tubular collar 184. The spring 170 is partially compressed in its rest state (i.e. when the movable light guide 142 is at its extended configuration shown in Figure 7C). In this way, the movable light guide 142 is normally biased in a direction away from the fixed light guide 150, that is, away from the light source 163 or light detector 165 as the case may be and, importantly, towards the animal body that is the subject of investigation.

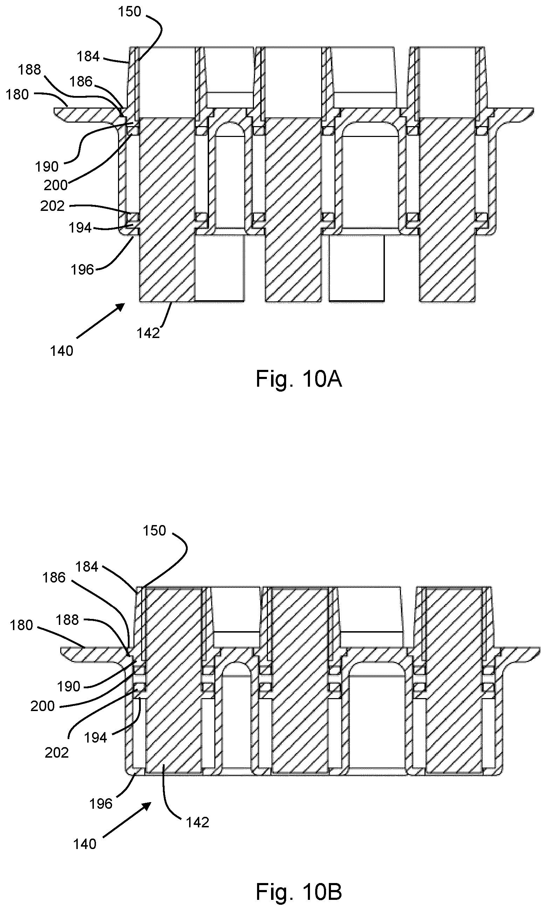

Alternatives to using springs for the biasing arrangement to bias the movable light guide(s) 142 towards the animal body are possible. For example, Figures 10A and 10B show cross-sectional views of another example of a light guide module according to the present disclosure in the first (extended) configuration and the second (retracted) configuration respectively. Parts that are the same as for the first example have the same reference numerals and will not be further described here. Not all reference numerals are repeated in Figures 10A and 10B for reasons of clarity.

In the example of Figures 10A and 10B, there is no tubular collar located within the elongate tubular collar 184 and within which the distal end of the movable light guide 142 is fixed. Rather, in this example, the movable light guide 142 itself has the external flange 194 around its periphery. Such an arrangement may be used in any of the examples described herein. This arrangement helps to keep down the parts count and can simplify assembly. Similarly to the previous example, the external flange 194 of the movable light guide 142 bears against the internally facing flange 196 at the lower part of the through hole 182 to retain the movable light guide 142 in the through hole 182 when the movable light guide 142 moves to the most extended position (downwards in Figure 10A).

Whereas the previous example of Figures 7C and 8C has a spring located in the cylindrical space between the movable light guide 142 and the lower part of the elongate tubular collar 184, the biasing arrangement of the example of Figures 10A and 10B is provided by two (or more) permanent magnets which are arranged to repel each other. In particular, referring to Figures 10A and 10B, at least one permanent magnet 200 is provided at the upper (proximal) part of the cylindrical space between the movable light guide 142 and the lower part of the elongate tubular collar 184, and at least one permanent magnet 202 is provided at the lower (distal) part of the cylindrical space between the movable light guide 142 and the lower part of the elongate tubular collar 184. The upper magnet 200 may be fixed to the internal flange 190 of the elongate tubular collar 184 and/or to the upper part of the adjacent wall of the module 140. The lower magnet 202 likewise may be fixed to the external flange 194 of the movable light guide 142 and/or to a lower part of the movable light guide 142. The magnets 200, 202 are arranged so that like poles (i.e. N-N or S-S) face each other so that the magnets 200, 202 repel each other. As such, the repelling action of the opposed magnets 200, 202 biases the movable light guide 142 away from the fixed light guide 150, that is, to the extended configuration and towards the animal body in use.

In the examples shown in Figure 7C and Figure 8C and in Figures 10A and 10B, the movable light guides 142 are solid. The solid light guides 142 may be formed of for example a single material. Suitable materials include for example injection moulded plastics, including for example polypropylene, polyamide (nylon), PMMA (poly(methyl methacrylate)), etc. The solid light guides 142 may alternatively be formed of a number of layers, for example a core layer and an outer layer having different refractive indices. The solid light guides 142 may have an outer coating which is formed of an opaque material to prevent light from outside the light guide 142 passing into the light guide 142. The outer coating also prevents light transmission from within the light guide 142 to the outside of the light guide 142. Each of the solid light guides 142 may be formed of for example a single optical fibre (of one or more layers) or a bundle of optical fibres (each being of one or more layers). Further, when present, the second tubular collar 192 may be present only for mechanical purposes, in order to provide mechanical support and protection for the movable light guide 142 contained within. Alternatively or additionally, the second tubular collar 192 may be present for optical purposes and may in effect be part of an overall light guide provided in part by the movable light guide 142. For example, the second tubular collar 192 may have an internal reflective surface (e.g. by having a silver or gold or similar coating) to perform a light guiding function. In this case, the second tubular collar 192 may be regarded as a functional outer part of the overall light guide, and the movable light guide 142 may be regarded as the inner part of the overall light guide provided by the combination of the second tubular collar 192 and central part 142 contained within.

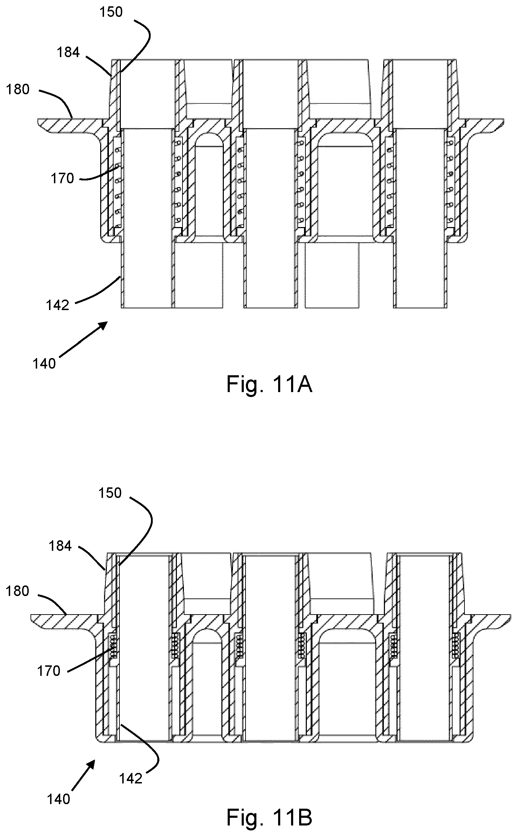

Another example of a light guide module according to the present disclosure is shown schematically in the cross-sectional views of Figures 11A and 11B, which correspond generally to the cross-sectional views of Figures 7C and 8C. Parts that are the same as for the examples of Figures 7C and 8C have the same reference numerals and will not be further described here. Not all reference numerals are repeated in Figures 11A and 11B for reasons of clarity.

Compared to the example of Figures 7C and 8C and Figures 10A and 10B, in the example of Figures 11A and 11B, instead of being solid, one or more or all of the movable light guides 142 are hollow tube light guides 142. These movable hollow light guides 142 are arranged to slide back and forth within the fixed hollow light guides 150. As for the previous examples having solid movable light guides, in this example as the movable hollow light guide 142 moves back and forth (for example, as the movable hollow light guides 142 are moved over the subject's body), the movable hollow light guide 142 moves back and forth within the fixed hollow tube light guide 150, in a reciprocating or telescopic manner. In the case that the movable hollow light guide 142 is for a light source 163, when the movable hollow light guide 142 moves away from the light source 163 (down the hollow fixed tube light guide 150 in Figure 11A), any light emitted by the light source 163 towards the movable hollow light guide 142 that may otherwise be lost is reflected by the interior surface of the hollow fixed tube light guide 150 towards the movable hollow light guide 142. Likewise, in the case that the movable hollow light guide 142 is for a light detector 165, when the movable hollow light guide 142 moves away from the light detector 165 (down the hollow fixed tube light guide 150 in Figure 11A), any light exiting the movable hollow light guide 142 that may otherwise be lost is reflected by the interior surface of the hollow fixed tube light guide 150 towards the light detector 165. This maximises the amount of light passing to and from the movable hollow light guide 142 and the light source 163 and light detector 165 respectively, even though the movable hollow light guide 142 has moved away to be remote from the light source 163 and light detector 165 as the case may be. This helps achieve a good signal to noise ratio.

An advantage of the movable light guide 142 being hollow, in addition to the fixed light guide 150 being hollow, is that this avoids an interface between a solid material and air (as occurs for light passing between the solid movable light guides 142 and the hollow fixed light guides 150 of the examples described above). This reduces loss of light that occurs due to back reflections as light passes between a solid medium and air, again improving the signal-to-noise ratio.

It may be noted that the example of Figures 11A and 11B is illustrated as using a spring for a biasing arrangement to bias the movable light guides 142 to the extended configuration, like the example of Figures 7C and 8C. In another example having hollow movable and fixed light guides 142, 150, pairs of mutually repellent magnets may be used, as described for the example of Figures 10A and 10B.

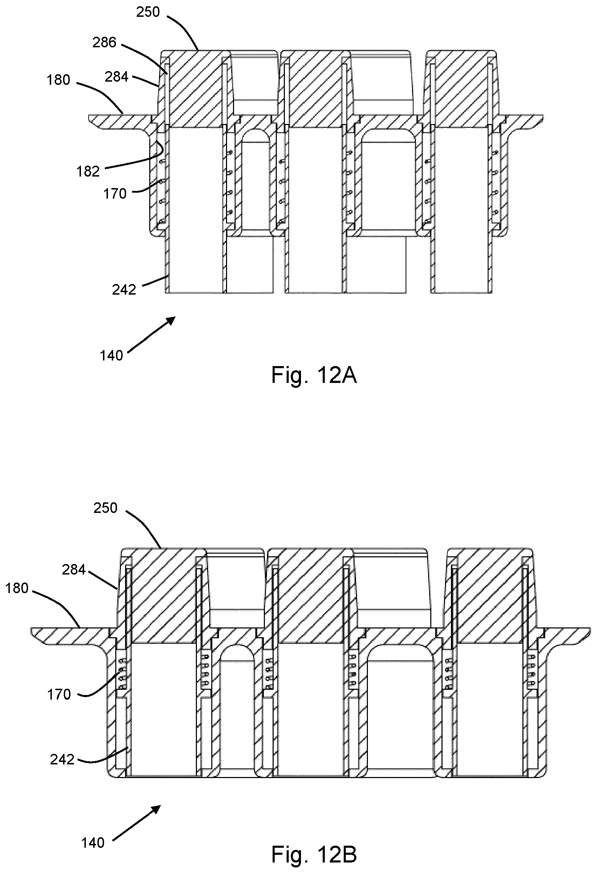

Another example of a light module according to the present disclosure is shown schematically in the cross-sectional views of Figures 12A and 12B, which correspond generally to the cross-sectional views of Figures 7C and 8C. Parts that are the same as for the previous examples, or that correspond, have the same reference numerals and will not be further described here. Not all reference numerals are repeated in Figures 12A and 12B for reasons of clarity.

The principal difference of the example of Figures 12A and 12B compared to the example of Figures 7C and 8C is that in this example, the movable light guide 242, which is at the distal (lower) end, near the subject in use, is in the form of a hollow tube, having a reflective interior, which passes over the fixed light guide 250 at the proximal (upper) end as the movable light guide 242 moves back and forth. This is in essence the reverse of the arrangement shown in Figures 7C and 8C, where the fixed light guide 150, at the proximal (upper) end, is a larger hollow tube which receives the movable light guide 142 as the movable light guide 142 moves back and forth.

In the example of Figures 12A and 12B, a short tubular collar 284 is located in the upper part of each through hole 182 of the base portion 180. The fixed light guide 250 is fixed to the short tubular collar 284. A cylindrical space 286 is defined between the adjacent walls of the short tubular collar 284 and the fixed light guide 250. The cylindrical space 286 receives the movable light guide 242 as the movable light guide 242 moves up into the retracted configuration, where the movable light guide 242 moves over or around the fixed light guide 250.

In the example of Figures 12A and 12B, the biasing arrangement is illustrated as being provided by a compression spring, similarly to the example of Figures 7C and 8C. In a variant of this, the biasing arrangement may be provided by one or more pairs of mutually repellent magnets, similarly to the example of Figures 10A and 10B.

In addition, in the example of Figures 12A and 12B, the fixed light guide 250 is illustrated as being a solid light guide, similarly to the example of Figures 7C and 8C (albeit with the relative locations of the solid and hollow light guides being reversed in this example). In a variant of this, in addition to the movable light guide 242 being a hollow tube, the fixed light guide 250 may be a hollow light guide. This is similar to the arrangement of two hollow light guides described above with reference to Figures 11A and 11B though with the larger hollow tube light guide 242 which receives the narrower light guide 250 being located at the distal (lower) end in this variant.

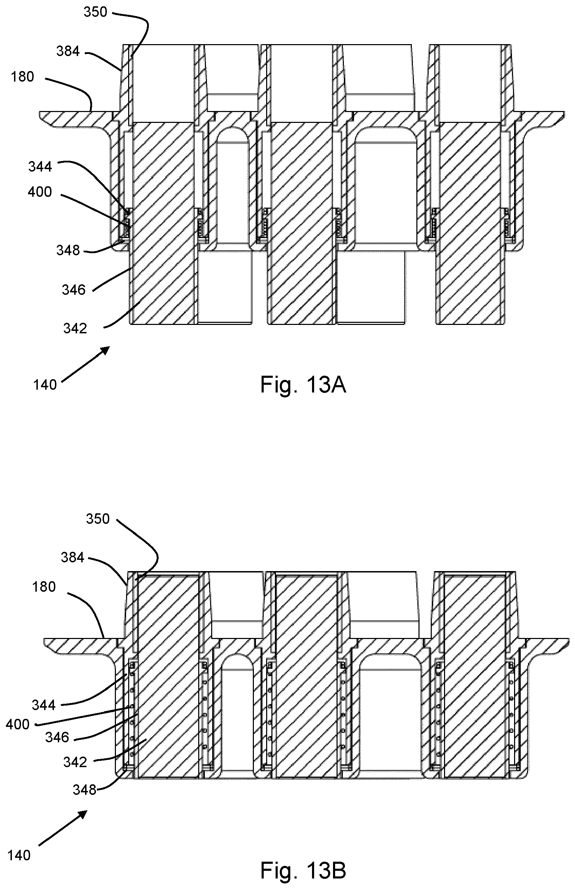

In the examples of Figures 7C and 8C, Figures 11A and 11B and Figures 12A and 12B, the biasing arrangement for each pair of fixed light guide and movable light guide (whether one hollow light guide and one solid light guide, and whether the solid light guide is at the distal end or the proximal end, or both the fixed and movable light guide guides are hollow) is illustrated as being provided by a compression spring. As another example, the biasing arrangement for each pair of fixed light guide and movable light guide may be an extension spring. An example of this is illustrated schematically in Figures 13A and 13B. It may be noted that in this example as illustrated, for each pair of fixed light guide and movable light guide, there is a hollow fixed light guide at the proximal end and a solid movable light guide at the distal end. However, all the variations discussed above apply equally, such that for example the movable light guide may be hollow and the fixed light guide may be solid, or both the light guides may be hollow.

Referring to Figures 13A and 13B, in this example, the movable light guide 342, which is at the distal (lower) end, near the subject in use, has an external retainer 344, which may be in the form of one or more projecting lugs or an annular ridge or the like. The retainer 344 may be integrally formed with the movable light guide 342 or may be provided on an external sleeve or collar 346 in which the movable light guide 342 is fixed. The retainer 344 is part way along the length of the movable light guide 342 in this example. A first end of an extension spring 400 is connected to the retainer 344 of the movable light guide 342 to be held by the retainer 344. A second end of the extension spring 400 is held by a second retainer 348 which is fixed relative to the planar base portion 180 of the light guide module 140. In this example, the second retainer 348 is fixed to or integral with an elongate tubular collar 384 which is located in each through hole 182 to extend therethrough and in which the movable light guide 342 moves and in which the fixed light guide 350 is mounted. It will be understood that different arrangements for retaining the first and second ends of the extension spring 400 are possible.

In the rest state illustrated in Fig. 13A, (i.e. when the movable light guide 342 is at its extended configuration), the extension spring 400 is partly extended to be under tension. In this way, the movable light guide 342 is normally biased in a direction away from the fixed light guide 350, that is, away from the light source or light detector as the case may be and, importantly, towards the animal body that is the subject of investigation.

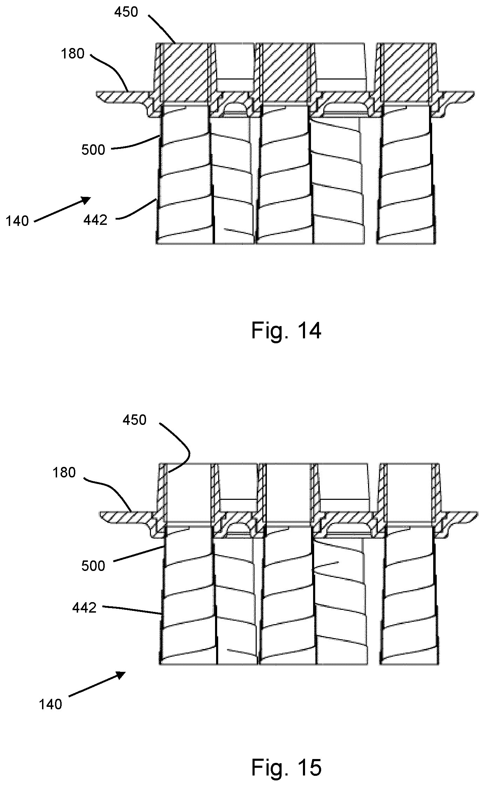

Other examples for the biasing arrangement and the movable and fixed light guides are illustrated schematically in Figures 14 to 17. In each of these examples, the biasing arrangement for biasing the movable light guide to the extended configuration is provided by a volute spring. In some examples, the movable light guide is provided by the volute spring and the fixed light guide is provided by a solid or hollow light guide, which is typically cylindrical. In one of the examples, the movable and the fixed light guide are provided by respective coils of the volute spring. An advantage of these arrangements is that a separate biasing arrangement, provided by separate compression or extension springs or pairs of magnets, etc., is not required.

Referring first to Figure 14, for each pair of light guides, the light guide module 140 has a fixed light guide 450 at the proximal (upper) end and a movable light guide 442 at the distal (lower end). The fixed light guide 450 is a solid light guide. Notably, the movable light guide 442 is provided by a volute spring 500 in this example.

As known per se, a volute spring is a compression spring having a number of coils. Under compression, the coils slide past each other, thus enabling the spring to be compressed to a very short length, with the resilience of the material of the volute spring biasing the spring to an extended configuration. Volute springs are often in the form of a cone, and may also be referred to as conical springs. In some cases however, a volute coil may have a parabolic profile. In the present examples, the volute spring 500 is typically a right cone (i.e. a cone for which the axis passes through the centre of the base at right angles to its plane) and typically has a circular cross-sectional shape.

The volute spring 500 is fixed at its proximal end to the main body of the light guide module 140. The proximal end of the volute spring 500 is adjacent to the facing end of the fixed light guide 450. The other, distal, end of the volute spring 500 projects out of the light guide module 140 and can move back and forth. Accordingly, as the light guide module 140 is moved over a subject, the volute spring 500 can compress and extend to accommodate recesses and ridges and the like in the scalp or other body part of the subject.

The volute spring 500 has a reflective interior, by for example being coated with or formed of a reflective material. In this example, the volute spring 500 therefore provides the movable light guide, at the distal end of the light guide module 140, as well as the biasing arrangement.

Referring to Figure 15, in this example, again for each pair of light guides, the light guide module 140 has a fixed light guide 450 at the proximal (upper) end and a movable light guide 442 at the distal (lower) end. In this example, the movable light guide 442 is again provided by a volute spring 500 which has a reflective interior and which also provides the biasing arrangement. This example differs from the example of Figure 14 in that the fixed light guide 450 is a hollow light guide.

In each of the examples of Figures 14 and 15, the volute springs 500 are oriented so that the narrow end of the volute spring 500 is towards the proximal end (closest to the light sources and the light detectors respectively) and the wider end of the volute spring 500 is towards the distal end (closest to the subject in use).

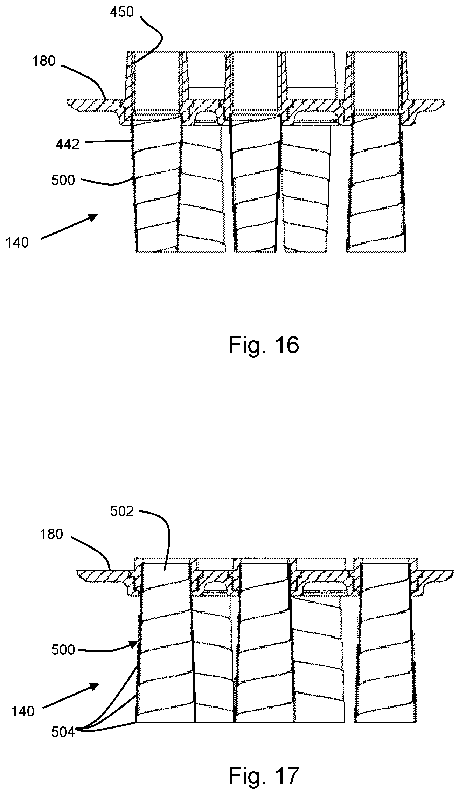

In another example shown schematically in Figure 16, one or more of the volute springs 500 are oriented so that the narrow end of the volute spring 500 is towards the proximal end and one or more of the volute springs 500 are oriented so that the wider end of the volute spring 500 is towards the proximal end. There may be particular advantages in orienting some volute springs 500 one way and orienting other volute springs 500 the other way. For example, it may be desirable if light travels along the interior of a volute spring 500 in a direction from the narrow end to the wider end. In this way, the light is less likely to be reflected at junctions between adjacent coils in the volute spring 500, given that narrower coils lie within the wider coils at their adjoining edges. Thus, in one example, the volute springs 500 are oriented so that the volute springs 500 that provide light guides for light sources are oriented so that the narrow end is at the proximal (upper) end, and the volute springs 500 that provide light guides for light detectors are oriented so that the wider end is at the proximal (upper) end.

In each of the examples of Figures 14 to 16, the movable light guide 442 is provided by the volute spring 500 and the fixed light guide 450 is provided by a solid or hollow light guide 450.

On the other hand, in the example of Figure 17, both the movable light guide and the fixed light guide are provided by respective coils of the volute spring 500. In particular, the volute spring 500 again has a reflective interior. Further, the proximal end of the volute spring 500 is fixed to the main body of the light guide module 140 such that the most proximal coil 502 of the volute spring 500 is fixed and provides the fixed light guide 502. On the other hand, one or more of the other coils 504 of the volute spring 500 are movable relative to the main body of the light guide module 140 such that these other coils 504 provide one or more movable light guides. An advantage of this arrangement is that a single piece (the single volute spring 500) provides both the fixed and the movable light guides and the biasing arrangement.

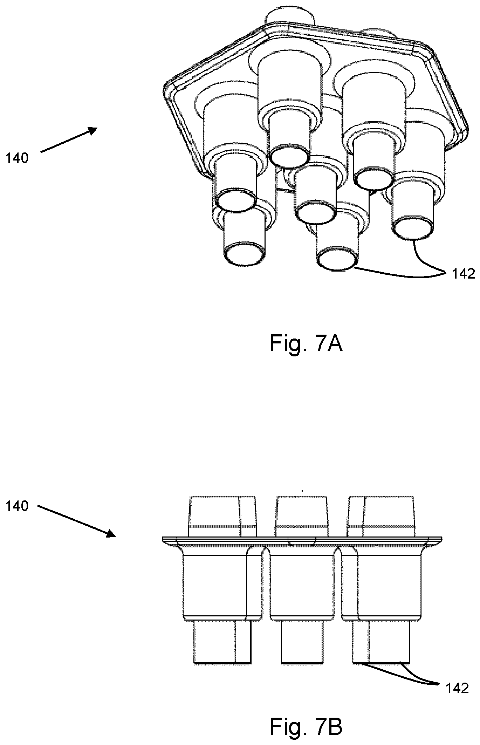

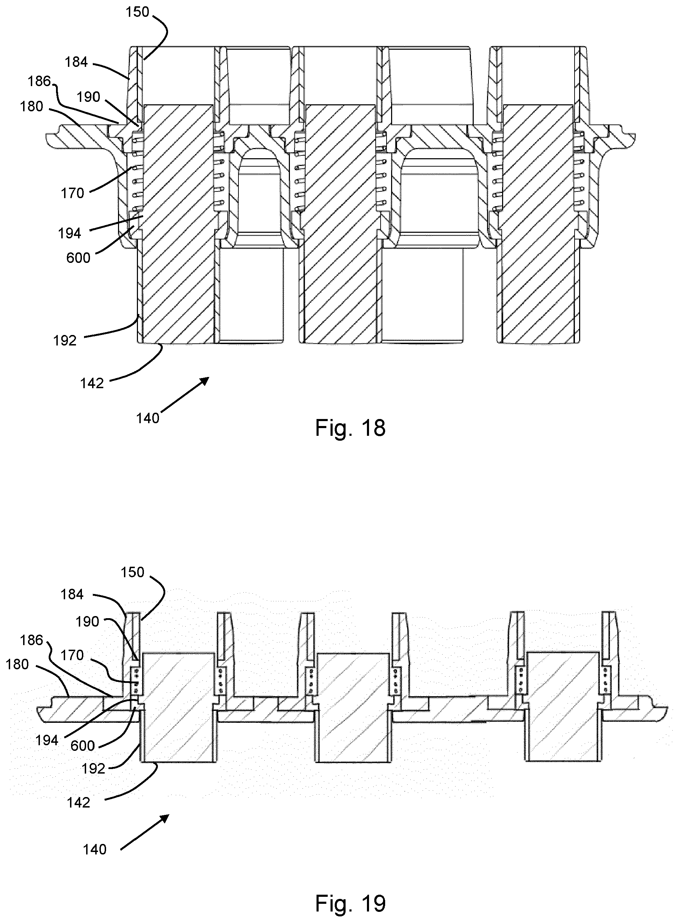

In any of the examples of the light guide module 140 above, it may be of advantage to provide one or more additional measures to prevent or at least reduce the amount of external or ambient light entering into the transmission path of light from the light source(s) to the subject and/or from the subject to the light detector(s). An example is shown schematically in Figure 18. The example of Figure 18 corresponds generally to the example of for example Figures 7 and 8 and the same reference numerals are used for the same or corresponding parts, though not all reference numerals are repeated for clarity. In these examples, each of the movable light guides 142 is solid and each of the fixed light guides 150 is a hollow tube, which is arranged coaxially with the respective movable light guide 142, and having respective springs 170 to bias the movable light guide 142 in a direction away from the fixed light guide 150. In the example of Figure 18, the spring 170 is located in the cylindrical space provided between the movable light guide 142 and the lower part of the elongate tubular collar 184 and between the movable light guide 142 and the downwardly projecting part of the base portion 180 of the light guide module 140. Further, in the example of Figure 18, the movable light guide 142 itself has the external flange 194, with the spring 170 being trapped between the external flange 194 of the movable light guide 142 and the internal flange 190 of the elongate tubular collar 184. However, the same principle may be used in one or more of the other examples described herein, which have different arrangements of the light guides and the biasing arrangement.

In the example of Figure 18, an opaque material 600 is located in the cylindrical space. In the example shown, the opaque material 600 is in the form of a single ring which wraps around the exterior of the movable light guide 142. Other arrangements for the opaque material 600 may be used, such as use of several parts of opaque material 600 to wrap around the exterior of the movable light guide 142. The opaque material 600 may be formed of for example black ABS (acrylonitrile butadiene styrene) or some other suitable opaque material. In the example shown, the opaque material 600 extends fully laterally across the cylindrical space at the lower end of the cylindrical space to fill the space at the lower end. The opaque material 600 acts as a blackout ring to prevent or at least reduce the amount of external or ambient light entering into light guides 142, 150, that is, into the transmission path of light from the light source(s) to the subject and/or from the subject to the light detector(s). This further helps to improve the signal-to-noise ratio.

Whilst in the example of Figure 18, the opaque material 600 is located at the lower end of the cylindrical space which is outside the movable light guide 142, similar opaque material 600 may be located additionally or alternatively part way up the cylindrical space and/or towards the top of the cylindrical space.

In each of the examples described above, the distal ends of the light guides that are closest to the subject in use (i.e. the end of those light guides that is remote from the light source or light detector respectively and that is therefore closest to the subject in use) extend a relatively long way from the generally planar base portion 180 of the light guide module 140. This is useful in many applications. However, in some applications, such as when the apparatus and therefore the light guide module 140 is used for study of a baby or infant's brain and is therefore used on a relatively small head, it can be better if the light guide module 140 is more low profile, that is, so that the overall vertical height of the light guide module 140 is less and particularly such that the distal ends of the light guides that are closest to the subject in use do not project far from the generally planar base portion 180 of the light guide module 140.

An example of a low profile light guide module 140 is shown schematically in Figure 19. The example of Figure 19 corresponds generally to the example of for example Figures 7 and 8 and Figure 18 and the same reference numerals are used for the same or corresponding parts, though not all reference numerals are repeated for clarity. In these examples, each of the movable light guides 142 is solid and each of the fixed light guides 150 is a hollow tube, which is arranged coaxially with the respective movable light guide 142, and having respective springs 170 to bias the movable light guide 142 in a direction away from the fixed light guide 150. However, the same low profile principle may be used in one or more of the other examples described herein, which have different arrangements of the light guides and the biasing arrangement.

Notably, in each of the examples described above, the biasing arrangement if provided is located on the side of the light guide module 140 that is closest to the subject in use. Referring for example to Figures 7c and 8c and Figure 18, the biasing arrangement, such as a spring 170, is located "below" the main plane of the generally planar base portion 180 of the light guide module 140.

In contrast, in the low profile light guide module 140 of Figure 19, the biasing arrangement, here a spring 170, for each movable light guide 142 is located "above" the main plane of the generally planar base portion 180 of the light guide module 140, that is, on the side of the light guide module 140 that is furthest from the subject in use. The biasing arrangement, here a spring 170, is located within the lower portion of the elongate tubular collar 184. This allows the overall vertical height of the light guide module 140 to be significantly reduced compared to the examples described above.

This is achieved in this example first by locating the internal flange 190 of the elongate tubular collar 184 of the base portion 180 above the external flange 186 of the elongate tubular collar 184 (that is, on the side of the external flange 186 of the elongate tubular collar 184 that is furthest from the subject in use). In addition, the lower (movable) light guide 142 is relatively shorter and the external flange 194 on the lower (movable) light guide 142 is located relatively further up. In particular, in this example, the external flange 194 on the lower (movable) light guide 142 is generally coplanar with the generally planar base portion 180 of the light guide module 140 in the rest position shown in Figure 19.

As in examples described above, the spring 170 is located in the cylindrical space between the movable light guide 142 and the lower part of the elongate tubular collar 184, and is trapped between the external flange 194 of the movable light guide 142 and the internal flange 190 of the elongate tubular collar 184. But here, the spring 170 is located "above" the main plane of the generally planar base portion 180, allowing the movable light guide 142 to be relatively shorter and the overall vertical height of the light guide module 140 to be small.

It is mentioned again that the same low profile principle, with the biasing arrangement "above" the main plane of the generally planar base portion 180 of the light guide module 140, may be used in one or more of the other examples described herein, which have different arrangements of the light guides and the biasing arrangement.

For completeness, it is noted that the example of Figure 19 is also shown as having opaque material 600 which acts as a blackout ring to prevent or at least reduce the amount of external or ambient light entering into light guides 142, 150, as described above with reference to Figure 18.

In summary, examples described herein provide sets of two (or more) light guides for one or more of each light source and light detector of the measuring apparatus. At least one of the light guides is a hollow light guide having a reflective interior into which the other light guide relatively moves. This use of a hollow light guide having a reflective interior accommodates relative movement between the light guides whilst maintaining a good transfer of light. This allows the distal light guide to move to come into contact or at least be closer to the subject animal body in use whilst minimising light loss and minimising admission of ambient light.

The examples described herein are to be understood as illustrative examples. Further embodiments and examples are envisaged. Any feature described in relation to any one example or embodiment may be used alone or in combination with other features. In addition, any feature described in relation to any one example or embodiment may also be used in combination with one or more features of any other of the examples or embodiments, or any combination of any other of the examples or embodiments. Furthermore, equivalents and modifications not described herein may also be employed within the scope of the invention, which is defined in the claims.

## Claims

1. A light guide module (140) for use with an apparatus which fits to an animal body for measuring an optical property of a medium in the animal body, the light guide module (140) comprising:
a support member;
a first light guide (142) supported in the support member; and
a second light guide (150) supported in the support member;
wherein one of the first light guide (142) and the second light guide (150) is a fixed light guide which is arranged to be fixed relative to the support member, and wherein the other of the first light guide (142) and the second light guide (150) is a movable light guide which is arranged to be movable back and forth relative to the support member between an extended position and a retracted position;
wherein the second light guide (150) is arranged coaxially with the first light guide (142) so that light can pass between the first light guide (142) and the second light guide (150);
**characterised in that** at least one of the fixed light guide and the movable light guide is a hollow tube light guide having a hollow interior which receives the other light guide;
the interior of said hollow tube light guide being reflective such that when the movable light guide is in the extended position, at least one of: (i) light entering an end of said hollow tube light guide can be internally reflected by the interior of said hollow tube light guide into an end of the other light guide that is facing the other end of said hollow tube light guide, and (ii) light exiting said end of the other light guide that is facing the other end of said hollow tube light guide can be internally reflected by the interior of said hollow tube light guide towards said end of said hollow tube light guide.

2. A light guide module (140) according to claim 1, wherein the fit between the hollow interior of said hollow tube light guide which receives the other light guide and the other light guide is a clearance fit.

3. A light guide module (140) according to claim 1 or claim 2, wherein said hollow tube light guide is the fixed light guide and is fixed to and supported by the support member or integral with the support member so as to be fixed against movement relative to the support member.

4. A light guide module (140) according to any of claims 1 to 3, wherein said hollow tube light guide is the movable light guide.

5. A light guide module (140) according to any of claims 1 to 4, wherein one of the light guides (142, 150) is said hollow tube light guide and the other light guide is a solid optical fibre or bundle of solid optical fibres.

6. A light guide module (140) according to any of claims 1 to 5, wherein both the first light guide (142) and the second light guide (150) are hollow tube light guides each having a reflective interior, wherein one of the hollow light guides is the movable light guide which is movable in and out of the hollow interior of the other, fixed, light guide.

7. A light guide module (140) according to any of claims 1 to 6, comprising a biasing arrangement to bias the movable light guide in a direction away from the fixed light guide.

8. A light guide module (140) according to any of claims 1 to 7, wherein the biasing arrangement comprises a spring (170; 400) arranged to bias the movable light guide in a direction away from the fixed light guide.

9. A light guide module (140) according to any of claims 1 to 7, wherein the biasing arrangement comprises a first magnet (202) fixed in relation to the movable light guide and a second magnet (200) fixed in relation to the fixed light guide, the magnets (200, 202) being arranged to repel each other so as to bias the movable light guide in a direction away from the fixed light guide.

10. A light guide module (140) according to any of claims 1 to 7, wherein the biasing arrangement is provided by a volute spring (500) having a plurality of coils, at least one of the first light guide (142) and the second light guide (150) being provided by at least one coil of the volute spring (500).

11. A light guide module (140) according to claim 10, wherein the first light guide (142) and the second light guide (150) are provided by respective coils of the volute spring (500).

12. A light guide module (140) according to any of claims 1 to 11, the light guide module (140) comprising:
plural sets of first and second light guides (142, 150) supported in the support member;
wherein for each set of first and second light guides (142, 150):
one of the first light guide (142) and the second light guide (150) is a fixed light guide which is arranged to be fixed relative to the support member, and wherein the other of the first light guide (142) and the second light guide (150) is a movable light guide which is arranged to be movable back and forth relative to the support member between an extended position and a retracted position;
the second light guide (150) is arranged coaxially with the first light guide (142) so that light can pass between the first light guide (142) and the second light guide (150);
at least one of the fixed light guide and the movable light guide is a hollow tube light guide having a hollow interior which receives the other light guide; and
the interior of said hollow tube light guide being reflective such that when the movable light guide is in the extended position, at least one of: (i) light entering an end of said hollow tube light guide can be internally reflected by the interior of said hollow tube light guide into an end of the other light guide that is facing the other end of said hollow tube light guide, and (ii) light exiting said end of the other light guide that is facing the other end of said hollow tube light guide can be internally reflected by the interior of said hollow tube light guide towards said end of said hollow tube light guide.

13. A light guide module (140) according to claim 12, wherein each of the movable light guides is movable back and forth relative to the support member independently of the other movable light guides.

14. An apparatus for fitting to an animal body for measuring an optical property of a medium in the animal body, the apparatus comprising:
at least one light source (163) for emitting light towards an animal body to which the measuring apparatus is fitted in use;
at least one light detector (165) for detecting light returning from said animal body; and
a light guide module (140) according to any of claims 1 to 13.

## Patentansprüche

1. Lichtleitermodul (140) zur Verwendung mit einer Vorrichtung, die an einem Tierkörper angebracht wird, um eine optische Eigenschaft eines Mediums in dem Tierkörper zu messen, wobei das Lichtleitermodul (140) umfasst:
ein Tragelement;
einen ersten Lichtleiter (142), der in dem Tragelement gelagert ist; und
einen zweiten Lichtleiter (150), der in dem Tragelement gelagert ist;
wobei
einer des ersten Lichtleiters (142) und des zweiten Lichtleiters (150) ein fixierter Lichtleiter ist, der so angeordnet ist, dass er in Bezug auf das Tragelement fixiert ist, und wobei
der andere des ersten Lichtleiters (142) und des zweiten Lichtleiters (150) ein bewegbarer Lichtleiter ist, der so angeordnet ist, dass er in Bezug auf das Tragelement zwischen einer ausgefahrenen Position und einer eingefahrenen Position hin und her bewegbar ist;
wobei der zweite Lichtleiter (150) koaxial mit dem ersten Lichtleiter (142) angeordnet ist, sodass Licht zwischen dem ersten Lichtleiter (142) und dem zweiten Lichtleiter (150) hindurchlaufen kann;
**dadurch gekennzeichnet, dass**
mindestens einer des fixierten Lichtleiters und des bewegbaren Lichtleiters ein Hohlrohr-Lichtleiter mit einem hohlen Innenraum ist, in dem der andere Lichtleiter aufgenommen ist;
der Innenraum des Hohlrohr-Lichtleiters reflektierend ist, sodass, wenn sich der bewegbare Lichtleiter in der ausgefahrenen Position befindet, mindestens eines vorliegt von: (i) in ein Ende des Hohlrohr-Lichtleiters eintretendes Licht kann von dem Innenraum des Hohlrohr-Lichtleiters in ein Ende des anderen Lichtleiters, das dem anderen Ende des Hohlraum-Lichtleiters zugewandt ist, intern reflektiert werden, und (ii) aus dem Ende des anderen Lichtleiters, das dem anderen Ende des Hohlraum-Lichtleiters zugewandt ist, austretendes Licht kann von dem Innenraum des Hohlrohr-Lichtleiters hin zu dem Ende des Hohlrohr-Lichtleiters intern reflektiert werden.

2. Lichtleitermodul (140) nach Anspruch 1, wobei die Passung zwischen dem hohlen Innenraum des Hohlrohr-Lichtleiters, das den anderen Lichtleiter aufnimmt, und dem anderen Lichtleiter eine Spielpassung ist.

3. Lichtleitermodul (140) nach Anspruch 1 oder Anspruch 2, wobei der Hohlrohr-Lichtleiter der fixierte Lichtleiter ist und an dem Tragelement fixiert und darin gelagert ist oder einstückig mit dem Tragelement ist, sodass er gegen eine Bewegung in Bezug auf das Tragelement fixiert ist.

4. Lichtleitermodul (140) nach einem der Ansprüche 1 bis 3, wobei der Hohlrohr-Lichtleiter der bewegbare Lichtleiter ist.

5. Lichtleitermodul (140) nach einem der Ansprüche 1 bis 4, wobei einer der Lichtleiter (142, 150) der Hohlrohr-Lichtleiter ist und der andere Lichtleiter eine massive optische Faser oder ein Bündel massiver optischer Fasern ist.

6. Lichtleitermodul (140) nach einem der Ansprüche 1 bis 5, wobei sowohl der erste Lichtleiter (142) als auch der zweite Lichtleiter (150) Hohlrohr-Lichtleiter sind, die jeweils einen reflektierenden Innenraum aufweisen, wobei einer der Hohlrohr-Lichtleiter der bewegbare Lichtleiter ist, der in den und aus dem hohlen Innenraum des anderen, fixierten Lichtleiters hinein- und herausbewegbar ist.

7. Lichtleitermodul (140) nach einem der Ansprüche 1 bis 6, umfassend eine Vorspannanordnung zum Vorspannen des bewegbaren Lichtleiters in einer Richtung weg von dem fixierten Lichtleiter.

8. Lichtleitermodul (140) nach einem der Ansprüche 1 bis 7, wobei die Vorspannanordnung eine Feder (170; 400) umfasst, die zum Vorspannen des bewegbaren Lichtleiters in einer Richtung weg von dem fixierten Lichtleiter angeordnet ist.

9. Lichtleitermodul (140) nach einem der Ansprüche 1 bis 7, wobei die Vorspannanordnung einen ersten Magneten (202) umfasst, der in Bezug auf den bewegbaren Lichtleiter fixiert ist, und einen zweiten Magneten (200), der in Bezug auf den fixierten Lichtleiter fixiert ist, wobei die Magneten (200, 202) so angeordnet sind, dass sie sich gegenseitig abstoßen, um den bewegbaren Lichtleiter in einer Richtung weg von dem fixierten Lichtleiter vorzuspannen.

10. Lichtleitermodul (140) nach einem der Ansprüche 1 bis 7, wobei die Vorspannanordnung durch eine Schneckenfeder (500) mit einer Mehrzahl von Windungen bereitgestellt ist, wobei zumindest einer des ersten Lichtleiters (142) und des zweiten Lichtleiters (150) durch mindestens eine Windung der Schneckenfeder (500) bereitgestellt ist.

11. Lichtleitermodul (140) nach Anspruch 10, wobei der erste Lichtleiter (142) und der zweite Lichtleiter (150) durch jeweilige Windungen der Schneckenfeder (500) bereitgestellt sind.

12. Lichtleitermodul (140) nach einem der Ansprüche 1 bis 11, wobei das Lichtleitermodul (140) umfasst:
mehrere Sätze von ersten und zweiten Lichtleitern (142, 150), die in dem Tragelement gelagert sind;
wobei für jeden Satz von ersten und zweiten Lichtleitern (142, 150):
einer des ersten Lichtleiters (142) und des zweiten Lichtleiters (150) ein fixierter Lichtleiter ist, der so angeordnet ist, dass er in Bezug auf das Tragelement fixiert ist, und wobei der andere des ersten Lichtleiters (142) und des zweiten Lichtleiters (150) ein bewegbarer Lichtleiter ist, der so angeordnet ist, dass er in Bezug auf das Tragelement zwischen einer ausgefahrenen Position und einer eingefahrenen Position hin und her bewegbar ist;
der zweite Lichtleiter (150) koaxial mit dem ersten Lichtleiter (142) angeordnet ist, sodass Licht zwischen dem ersten Lichtleiter (142) und dem zweiten Lichtleiter (150) hindurchlaufen kann;
mindestens einer des fixierten Lichtleiters und des bewegbaren Lichtleiters ein Hohlrohr-Lichtleiter mit einem hohlen Innenraum ist, in dem der andere Lichtleiter aufgenommen ist; und
der Innenraum des Hohlrohr-Lichtleiters reflektierend ist, sodass, wenn sich der bewegbare Lichtleiter in der ausgefahrenen Position befindet, mindestens eines vorliegt von: (i) in ein Ende des Hohlrohr-Lichtleiters eintretendes Licht kann von dem Innenraum des Hohlrohr-Lichtleiters in ein Ende des anderen Lichtleiters, das dem anderen Ende des Hohlraum-Lichtleiters zugewandt ist, intern reflektiert werden, und (ii) aus dem Ende des anderen Lichtleiters, das dem anderen Ende des Hohlraum-Lichtleiters zugewandt ist, austretendes Licht kann von dem Innenraum des Hohlrohr-Lichtleiters hin zu dem Ende des Hohlrohr-Lichtleiters intern reflektiert werden.

13. Lichtleitermodul (140) nach Anspruch 12, wobei jeder der bewegbaren Lichtleiter unabhängig von den anderen bewegbaren Lichtleitern in Bezug auf das Tragelement hin und her bewegbar ist.

14. Vorrichtung zum Anbringen an einem Tierkörper, um eine optische Eigenschaft eines Mediums in dem Tierkörper zu messen, wobei die Vorrichtung umfasst:
mindestens eine Lichtquelle (163) zum Aussenden von Licht hin zu einem Tierkörper, an dem die Messvorrichtung bei der Verwendung angebracht ist;
mindestens einen Lichtdetektor (165) zum Erfassen von Licht, das von dem Tierkörper zurückgestrahlt wird; und
ein Lichtleitermodul (140) nach einem der Ansprüche 1 bis 13.

## Revendications

1. Module de guidage de lumière (140) destiné à être utilisé avec un appareil destiné à être monté sur le corps d'un animal pour mesurer une propriété optique d'un milieu présent dans le corps de l'animal, le module de guidage de lumière (140) comprenant :
un élément de support ;
un premier guide de lumière (142) porté par l'élément de support ; et
un deuxième guide de lumière (150) porté par l'élément de support ;
dans lequel
l'un du premier guide de lumière (142) et du deuxième guide de lumière (150) est un guide de lumière fixe agencé pour être fixe par rapport à l'élément de support, et dans lequel l'autre du premier guide de lumière (142) et du deuxième guide de lumière (150) est un guide de lumière mobile qui est agencé pour pouvoir se déplacer en va-et-vient par rapport à l'élément de support entre une position déployée et une position rétractée ;
dans lequel le deuxième guide de lumière (150) est disposé coaxialement au premier guide de lumière (142) de sorte que de la lumière puisse passer entre le premier guide de lumière (142) et le deuxième guide de lumière (150) ;
c**aractérisé en ce que**
au moins l'un du guide de lumière fixe et du guide de lumière mobile est un guide de lumière tubulaire creux présentant un intérieur creux dans lequel est reçu l'autre guide de lumière ;
l'intérieur dudit guide de lumière tubulaire creux étant réfléchissant de sorte que, lorsque le guide de lumière mobile est dans la position déployée, au moins l'une des situations suivantes se produit : (i) de la lumière entrant par une extrémité dudit guide de lumière tubulaire creux peut subir une réflexion interne sur l'intérieur dudit guide de lumière tubulaire creux vers une extrémité de l'autre guide de lumière qui fait face à l'autre extrémité dudit guide de lumière tubulaire creux, et (ii) de la lumière sortant de ladite extrémité de l'autre guide de lumière qui fait face à l'autre extrémité dudit guide de lumière tubulaire creux peut subir une réflexion interne sur l'intérieur dudit guide de lumière tubulaire creux vers ladite extrémité dudit guide de lumière tubulaire creux.

2. Module de guidage de lumière (140) selon la revendication 1, dans lequel l'ajustement entre l'intérieur creux dudit guide de lumière tubulaire creux, dans lequel est reçu l'autre guide de lumière, et l'autre guide de lumière est un ajustement avec jeu.

3. Module de guidage de lumière (140) selon la revendication 1 ou la revendication 2, dans lequel ledit guide de lumière tubulaire creux est le guide de lumière fixe et est fixé à l'élément de support et porté par celui-ci, ou est solidaire de l'élément de support, de manière à être fixe par rapport à l'élément de support.

4. Module de guidage de lumière (140) selon l'une des revendications 1 à 3, dans lequel ledit guide de lumière tubulaire creux est le guide de lumière mobile.

5. Module de guidage de lumière (140) selon l'une des revendications 1 à 4, dans lequel l'un des guides de lumière (142, 150) est ledit guide de lumière tubulaire creux et l'autre guide de lumière est une fibre optique pleine ou un faisceau de fibres optiques pleines.

6. Module de guidage de lumière (140) selon l'une des revendications 1 à 5, dans lequel le premier guide de lumière (142) et le deuxième guide de lumière (150) sont des guides de lumière tubulaire creux présentant chacun un intérieur réfléchissant, l'un des guides de lumière creux étant le guide de lumière mobile et pouvant se déplacer vers l'intérieur et vers l'extérieur de l'intérieur creux de l'autre guide de lumière, fixe.

7. Module de guidage de lumière (140) selon l'une des revendications 1 à 6, comprenant un dispositif de sollicitation destiné à solliciter le guide de lumière mobile dans une direction s'éloignant du guide de lumière fixe.

8. Module de guidage de lumière (140) selon l'une des revendications 1 à 7, dans lequel le dispositif de sollicitation comprend un ressort (170 ; 400) agencé pour solliciter le guide de lumière mobile dans une direction s'éloignant du guide de lumière fixe.

9. Module de guidage de lumière (140) selon l'une des revendications 1 à 7, dans lequel le dispositif de sollicitation comprend un premier aimant (202) fixe par rapport au guide de lumière mobile et un deuxième aimant (200) fixe par rapport au guide de lumière fixe, les aimants (200, 202) étant agencés pour se repousser mutuellement de manière à solliciter le guide de lumière mobile dans une direction s'éloignant du guide de lumière fixe.

10. Module de guidage de lumière (140) selon l'une des revendications 1 à 7, dans lequel le dispositif de sollicitation est constitué d'un ressort en volute (500) comportant une pluralité de spires, au moins l'un du premier guide de lumière (142) et du deuxième guide de lumière (150) étant constitué d'au moins une spire du ressort en volute (500).

11. Module de guidage de lumière (140) selon la revendication 10, dans lequel le premier guide de lumière (142) et le deuxième guide de lumière (150) sont constitués de spires respectives du ressort en volute (500).

12. Module de guidage de lumière (140) selon l'une des revendications 1 à 11, le module de guidage de lumière (140) comprenant :
plusieurs ensembles comprenant chacun un premier guide de lumière et un deuxième guide de lumière (142, 150) portés par l'élément de support ;
dans lequel, pour chaque ensemble de premier et deuxième guides de lumière (142, 150) :
l'un du premier guide de lumière (142) et du deuxième guide de lumière (150) est un guide de lumière fixe agencé pour être fixe par rapport à l'élément de support, et dans lequel l'autre du premier guide de lumière (142) et du deuxième guide de lumière (150) est un guide de lumière mobile agencé pour pouvoir se déplacer en va-et-vient par rapport à l'élément de support entre une position déployée et une position rétractée ;
le deuxième guide de lumière (150) est disposé coaxialement au premier guide de lumière (142) de sorte que de la lumière puisse passer entre le premier guide de lumière (142) et le deuxième guide de lumière (150) ;
au moins l'un du guide de lumière fixe et du guide de lumière mobile est un guide de lumière tubulaire creux présentant un intérieur creux dans lequel est reçu l'autre guide de lumière ; et
l'intérieur dudit guide de lumière tubulaire creux étant réfléchissant de sorte que, lorsque le guide de lumière mobile est dans la position déployée, au moins l'une des situations suivantes se produit : (i) de la lumière entrant par une extrémité dudit guide de lumière tubulaire creux peut subir une réflexion interne sur l'intérieur dudit guide de lumière tubulaire creux vers une extrémité de l'autre guide de lumière qui fait face à l'autre extrémité dudit guide de lumière tubulaire creux, et (ii) de la lumière sortant de ladite extrémité de l'autre guide de lumière qui fait face à l'autre extrémité dudit guide de lumière tubulaire creux peut subir une réflexion interne sur l'intérieur dudit guide de lumière tubulaire creux vers ladite extrémité dudit guide de lumière tubulaire creux.

13. Module de guide de lumière (140) selon la revendication 12, dans lequel chacun des guides de lumière mobiles peut se déplacer en va-et-vient par rapport à l'élément de support indépendamment des autres guides de lumière mobiles.

14. Appareil destiné à être monté sur le corps d'un animal pour mesurer une propriété optique d'un milieu présent dans le corps de l'animal, l'appareil comprenant :
au moins une source lumineuse (163) destinée à émettre de la lumière vers le corps d'un animal sur lequel l'appareil de mesure est monté lors de son utilisation ;
au moins un détecteur de lumière (165) destiné à détecter la lumière revenant dudit corps d'animal ; et
un module de guidage de lumière (140) selon l'une des revendications 1 à 13.
